# EUROPEAN PATENT SPECIFICATION

(11) **EP 3 739 040 B1**
(45) Date of publication and mention of the grant of the patent: **05.07.2023**
(21) Application number: 19866888.1
(22) Date of filing: 27.09.2019
(51) Int. Cl.: C12N 5/10, A61K 35/34, A61K 35/545, A61L 27/38, A61L 27/40, A61P 9/00, A61P 43/00, C12N 5/077

(54) **SHEETING METHOD FOR PLURIPOTENT STEM CELL-DERIVED CELLS**
ABSTEIFUNGSVERFAHREN FÜR ZELLEN AUS PLURIPOTENTEN STAMMZELLEN
PROCÉDÉ DE MISE EN FEUILLE POUR DES CELLULES DÉRIVÉES DE CELLULES SOUCHES PLURIPOTENTES

(30) Priority: 27.09.2018 JP 2018182442
(43) Date of publication of application: 18.11.2020
(73) Proprietor: Osaka University, Suita-shi, Osaka 565-0871 (JP); Terumo Kabushiki Kaisha, Tokyo 151-0072 (JP)
(72) Inventor: SAWA, Yoshiki, Suita-shi, Osaka 565-0871 (JP); MIYAGAWA, Shigeru, Suita-shi, Osaka 565-0871 (JP); OYAMA, Kenji, Ashigarakami-gun, Kanagawa 259-0151 (JP); OHASHI, Fumiya, Ashigarakami-gun, Kanagawa 259-0151 (JP)
(74) Representative: Prüfer & Partner mbB Patentanwälte · Rechtsanwälte
(86) International application number: PCT/JP2019/038191
(87) International publication number: WO 2020/067438

(56) References cited:
- EP-A1- 3 208 328
- WO-A1-2007/088874
- WO-A1-2016/072519
- WO-A1-2016/076368
- WO-A1-2016/167332
- WO-A1-2017/010544
- WO-A1-2017/038562
- WO-A1-2020/067435
- WO-A1-2020/067436
- JP-A- 2012 500 021
- US-A1- 2018 153 155
- JUNJUN LI ET AL: "Human Pluripotent Stem Cell-Derived Cardiac Tissue-like Constructs for Repairing the Infarcted Myocardium", STEM CELL REPORTS, vol. 9, no. 5, 1 November 2017 (2017-11-01), pages 1546-1559, XP055469464, United States ISSN: 2213-6711, DOI: 10.1016/j.stemcr.2017.09.007
- HIDETOSHI MASUMOTO ET AL: "Human iPS cell-engineered cardiac tissue sheets with cardiomyocytes and vascular cells for cardiac regeneration", SCIENTIFIC REPORTS, vol. 4, no. 1, 22 October 2014 (2014-10-22), pages 1-7, XP055399630, DOI: 10.1038/srep06716
- MATSUURA, KATSUHISA et al.: "Creation of human cardiac cell sheets using pluripotent stem cells", Biochemical and Biophysical Research Communications, vol. 425, 25 July 2012 (2012-07-25), pages 321-327, XP055482020, DOI: 10.1016/j.bbrc.2012.07.089

## Description

### Technical Field

The present disclosure is a patent application, to which Article 19 of the Industrial Technology Enhancement Act is applied, relating to a research and development concerning Research Center Network for Realization of Regenerative Medicine, Centers for Clinical Application Research on Specific Disease/Organ (Type A), "Center for the development of myocardial regenerative treatments using iPS cells" sponsored by Japan Agency for Medical Research and Development on 2018. This patent application relates to a method for producing a graft containing cells, such as a sheet-shaped cell culture, from pluripotent stem cell-derived differentiation-induced cells, wherein said differentiation-induced cells are cardiomyocytes, and wherein this method comprises performing an anti-CD30 antibody treatment.

### Background Art

Adult cardiomyocytes have a poor self-replication competence. Thus, when cardiac muscle tissues suffer damages, it is extremely difficult to repair the damages. In recent years, attempts have been made to transplant a graft containing cardiomyocytes produced by cell engineering techniques to an affected part for repairing damaged cardiac muscle tissues (Patent Literature 1, Non Patent Literature 1). Recently attracting attention as a source of cardiomyocytes used for producing such a graft are pluripotent stem cell-derived cardiomyocytes such as embryonic stem cells (ES cells) and induced pluripotent stem cells (iPS cells), and production of a sheet-shaped cell culture containing such pluripotent stem cell-derived cardiomyocytes and therapy experiments in animals have been attempted (Non Patent Literature 2 and 3). However, the development of the sheet-shaped cell culture containing such pluripotent stem cell-derived cardiomyocytes has just started, and functional properties of the cell culture and factors that affects the functional properties are still largely unknown.

Non Patent Literature 4 discloses obtaining cardiac tissue-like constructs by cultivating high-purity cardiomyocytes on nanofibers.

Non Patent Literature 5 discloses the generation of cardiovascular cell sheets from human induced pluripotent stem cells in order to realize cardiac regeneration, comprising a step of using supplementation of vascular endothelial cell growth factor for additional induction of vascular cells.

When cells used for the purpose of transplantation to a living body are differentiated from pluripotent stem cells by inducing the differentiation, in the obtained cell population, stem cells in an undifferentiated state having tumorigenic potential are present, and therefore the presence of remaining undifferentiated cells is a big problem. Thus, various method for removing stem cells in an undifferentiated state from desired cells after the desired cells are differentiated from pluripotent stem cells by inducing the differentiation have been investigated (Patent Literature 2 to 4).

Patent Literature 5 discloses a method for cryopreservation of cardiocytes derived from pluripotent or mesenchymal stem cells. Particularly, it also discloses removing of cell aggregates by a strainer.

Patent Literature 6 describes dissociation of cells by using a filter.

Patent Literature 7 discloses a method for producing a graft from differentiation-induced cells derived from a pluripotent stem cell, comprising the steps of removing undifferentiated cells, a step of optionally freezing a cell population and then thawing it; and a step of filtering the thawed cells.

Patent Literature 8 discloses a method for producing a sheet-shaped cell culture, comprising removing dead cells from the cell population and seeding a cell population containing sheet-forming cells on a culture substrate to form a sheet, or removing at least one kind of undifferentiated cell in a cell population, cryopreserving the cell population, thawing it and filtering it, and seeding the filtered cell population on a culture substrate.

### Citation List

### Patent Literature

Patent Literature 1: JP 2007-528755 A
Patent Literature 2: WO 2017/038562
Patent Literature 3: WO 2016/072519
Patent Literature 4: WO 2007/088874
Patent Literature 5: US 2018/153155
Patent Literature 6: EP 3 208 328
Patent Literature 7: WO 2020/067436
Patent Literature 8: WO 2020/067435

### Non Patent Literature

Non Patent Literature 1: Shimizu et al., Circ Res. 2002 Feb 22; 90(3): e40-e48
Non Patent Literature 2: Matsuura et al., Biomaterials. 2011 Oct; 32(30): 7355-62
Non Patent Literature 3: Kawamura et al., Circulation. 2012 Sep 11; 126(11 Suppl 1): S29-37
Non Patent Literature 4: Junjun Li et al., Stem Cell Reports. 2017 Nov 1; 9(5): 1546-59
Non Patent Literature 5: Hidetoshi Masumoto et al., Scientific Reports. 4(1): 2014 Oct 22: 1-7

### Summary of Invention

### Technical Problem

An object of the present disclosure is to provide a method for producing a high-quality graft such as a sheet-shaped cell culture from pluripotent stem cell-derived differentiation-induced cells.

### Solution to Problem

When cells differentiated from pluripotent stem cells by inducing the differentiation are used in clinical application, there are various requirements that are not necessary in basic research. For example, in preparation of a cell population for transplantation to a living body, all materials including raw materials and reagents used for the preparation should be applicable to transplantation purposes, that is, should be xeno-free. It is required for a cell population that has been differentiated by inducing the differentiation and has been prepared for transplantation to a living body to contain a minimum amount of remaining undifferentiated cells, and be capable of being cryopreserved.

The present inventors have made investigations on sheet-shaped cell cultures for transplantation to a living body using pluripotent stem cell-derived cardiomyocytes. In the investigations, the present inventors faced a new problem that, when a sheet-shaped cell culture applicable to clinical application is to be produced, it is difficult to produce a high-quality sheet-shaped cell culture using conventional methods. Pursuing investigations to solve the problem, the present inventors have obtained new findings that in a step of performing removal of undifferentiated cells for use in clinical application or performing cryopreservation, viability of cells are decreased, which results in the difficulty in producing the sheet-shaped cell culture.

Making further investigations based on the findings, the present inventors have found that by performing graft-forming culture under conditions that are different from those of previously known graft-forming method, a high-quality graft such as a sheet-shaped cell culture that is applicable to clinical application can be produced. As a result of still further investigations, the present inventors have made the present invention.

That is, the present invention provides a method for producing a sheet-shaped cell culture as defined in claim 1. Preferred embodiments of the method are set forth in dependent claims 2 to 9.

### Advantageous Effects of Invention

According to the present invention, a graft, such as a sheet-shaped cell culture, of higher quality than that in prior art can be produced with high efficiency from a cell population for clinical use differentiated from pluripotent stem cells by inducing the differentiation. In particular, the content of remaining undifferentiated cells can be minimized. Further, even when the cell population is cryopreserved, a graft such as a sheet-shaped cell culture can be produced without sacrificing quality. Thus, a graft that is extremely suitable for transplantation to a living body can be provided.

### Brief Description of Drawings

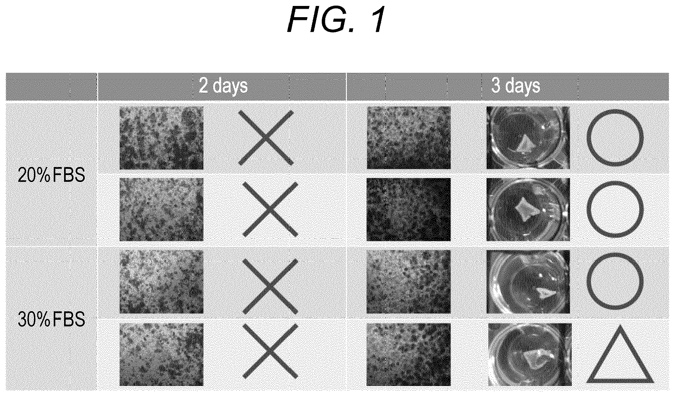

Fig. 1 is a table comparing appearances of sheet-shaped cell cultures when FBS concentrations of sheet culture media are changed in a production method in Production Example 2. It was confirmed that even in conditions in Production Example 2, sheets were formed by sheet-forming incubation at a FBS concentration of 20% for 3 days.

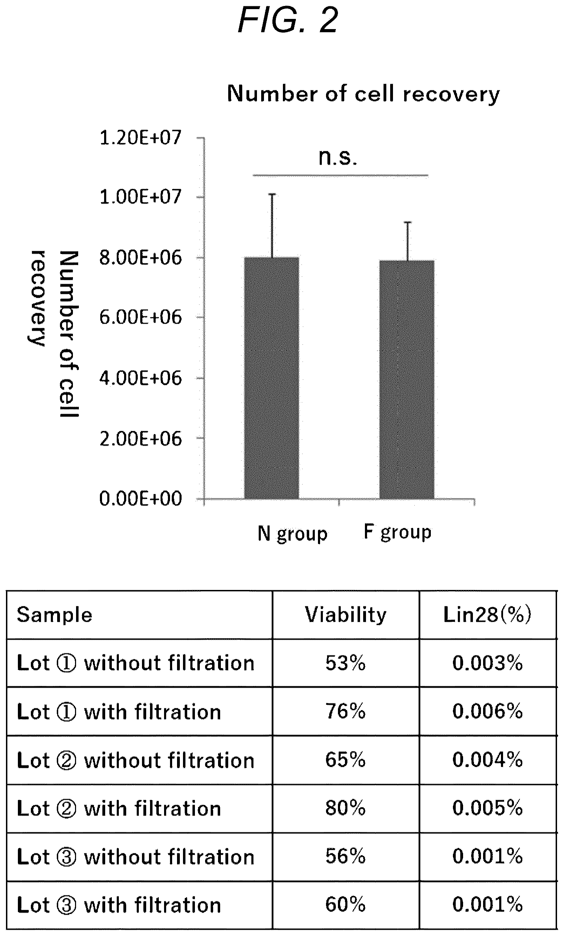

Fig. 2 shows the results of changes of cell populations caused by filtering treatment. The graph at the upper side is a graph showing differences in numbers of collected cells between a filtering-treated cell population and a cell population without filtering treatment. It is known that even when the filtering treatment was performed, there was almost no change in the number of collected cells. The table at the lower side is a table showing viabilities and Lin28 values in a filtering-treated cell population and a cell population without filtering treatment in each lot. It is known that when the filtering treatment was performed, as compared to the case without filtering treatment, the viability was increased, but there was almost no change in the Lin28 value.

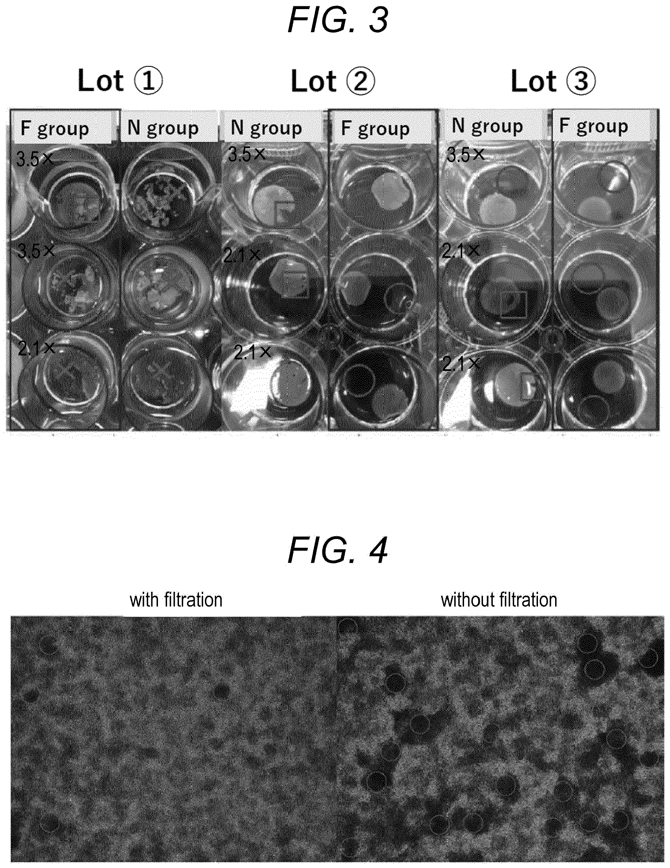

Fig. 3 is photographic images showing influence of a filtering treatment step on the qualities of sheet-shaped cell cultures. When the filtering treatment was performed, as compared to the case without filtering treatment, perforations and breakages were remarkably reduced. In the images, the region enclosed in a box is a portion in which a perforation or breakage was observed. The photograph marked with a circle shows that a sheet-shaped cell culture having no perforation or breakage was formed, and the photograph marked with x shows that no sheet was formed.
Fig. 4 is photographic images of observation of aggregates each in a field of view at a 10-fold magnification when a filtering-treated cell population or a cell population without filtering treatment was seeded on a culture substrate. In the images, the region enclosed in a circle is a portion in which an aggregate was observed. Without filtering, aggregates were observed in 19 portions. On the other hand, when filtering was performed, aggregates were observed in only 4 portions.

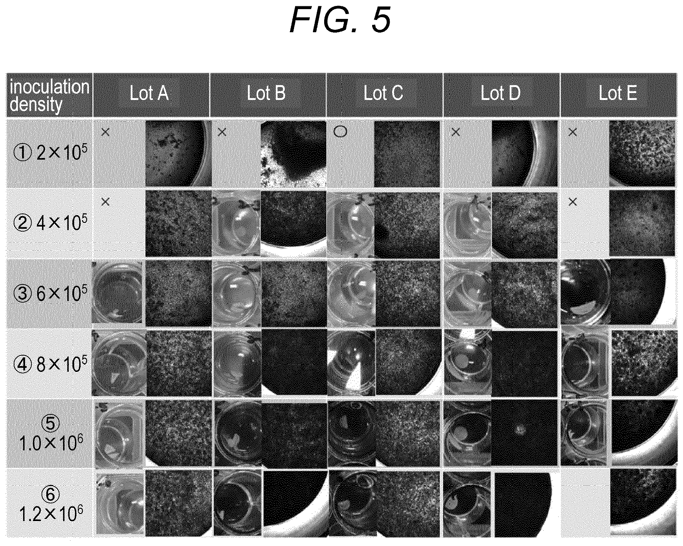

Fig. 5 is photographic images showing appearances at day 3 of culture when cardiomyocytes of different lots with different treatments were seeded on culture substrates at predetermined densities and performing sheet-forming incubation. At a seeding density of 2.0×10⁵ cells/cm², almost no sheet-forming occurred even at day 3 of the culture. On the other hand, at seeding densities of 6.0×10⁵ cells/cm² or more, in all lots, sheet-shaped cell cultures were obtained. At 4.0×10⁵ cells/cm², in lot E in which plane culture was not performed, sheet-forming was not quite sufficient, and a fragile sheet-shaped cell culture, as compared to other lots, was formed.

### Description of Embodiments

The present invention will be described in detail below.

Unless indicated otherwise herein, all technical and scientific terms have the same meanings as commonly understood by those skilled in the art. In the event that any inconsistency exists between the description of any publication referenced herein and the description of the present specification, the description of the present specification shall govern.

The references to methods of treatment, particularly in paragraphs [0061] to [0067] of this description, are to be interpreted as references to the compounds, pharmaceutical compositions and medicaments of the present invention for use in a method for treatment of the human (or animal) body by therapy (or for diagnosis).

In the present disclosure, the term "pluripotent stem cell" is a term well known in the art and means a cell having the ability to differentiate into cells of all lineages belonging to three germ layers, i.e., endoderm, mesoderm, and ectoderm. Non-limiting examples of the pluripotent stem cells include, for example, embryonic stem cells (ES cells), induced pluripotent stem cells (iPS cells), and the like. Herein, the iPS cells are cells that are induced by introducing genes and have differentiation omnipotence and self-replication competence. Usually, when pluripotent stem cells are differentiated into specific cells by inducing the differentiation, first, pluripotent stem cells are suspended and cultured to form aggregates of any of the above-described three germ layers (hereinafter, sometimes also referred to as "embryonic bodies"), and then cells forming the aggregates are differentiated into specific cells of interest by inducing the differentiation.

In the present disclosure, the term "pluripotent stem cell-derived differentiation-induced cells" refers to any cells that are subjected to differentiation-inducing treatment to differentiate from pluripotent stem cells into a specific type of cells. Non-limiting examples of the differentiation-induced cells include muscle-related cells such as cardiomyocytes and myoblast cells; nerve-related cells such as neuronal cells, oligodendrocytes, and dopamine-producing cells; retinal cells such as retinal pigment epithelial cells; hematopoiesis related cells such as blood cells and bone marrow cells; immune-related cells such as T cells, NK cells, NKT cells, dendritic cells, and B cells; cells that constitute organs such as liver cells, pancreatic beta cells, and renal cells ; chondrocytes; germ cells ; and the like; and also include progenitor cells and somatic stem cells that differentiate into the foregoing cells. Typical examples of the progenitor cells and the somatic stem cells include, for example, mesenchymal stem cells, multipotent cardiac progenitor cells, and unipotent cardiac progenitor cells in the cardiomyocytes; neural stem cells in the nerve-related cells; hematopoietic stem cells and lymphoid stem cell in the hematopoiesis related cells and the immune-related cells. Differentiation of pluripotent stem cells may be induced by using any method known in the art. For example, differentiation of pluripotent stem cells into cardiomyocytes may be induced according to methods described in Miki et al., Cell Stem Cell 16, 699-711, June 4, 2015 and WO 2014/185358.

Examples of a method of obtaining cardiomyocytes from human iPS cells include, for example, a method including the following steps:
(1) a step of maintaining and culturing established human iPS cells in a feeder cell-free medium (feeder free method);
(2) a step of forming embryonic bodies (embryonic bodies including mesodermal cells) from the obtained iPS cells;
(3) a step of culturing the obtained embryonic bodies in a culture solution containing activin A, bone morphogenetic protein (BMP) 4, and basic fibroblast growth factor (bFGF) ;
(4) a step of culturing the obtained embryonic bodies in a culture solution containing a Wnt inhibitor, a BMP4 inhibitor, and a TGFβ inhibitor; and
(5) a step of culturing the obtained embryonic bodies in a culture solution containing VEGF and bFGF.

In step (1), for example, as described in WO 2017/038562, using StemFit AK03 (Ajinomoto Co., Inc.) as a culture medium, iPS cells can be cultured and adapted on iMatrix 511 (Nippi, Incorporated), and can be maintained and cultured. In addition, as described in, for example, Nakagawa M., et al. A novel efficient feeder-free culture system for the derivation of human induced pluripotent stem cells. Sci Rep. 2014; 4: 3594, passage of iPS cells can be performed every 7 to 8 days in a single cell state using TrypLE (registered trademark) Select (Thermo Fisher Scientific). After the above-described steps (1) to (5), optionally (6) a step of purifying the obtained cardiomyocytes may be selectively performed. Purification of cardiomyocytes includes, as described in detail below, a method of reducing cells other than cardiomyocytes using a glucose free medium, a method of reducing undifferentiated cells using heat treatment as described in WO 2017/038562, and the like. In the method of the present invention, in step (A), removing of undifferentiated cells in a cell population containing pluripotent stem cell-derived differentiation-induced cells, which are cardiomyocytes, is carried out by performing one single operation being anti-CD30 antibody treatment, or by performing at least two different operations comprising anti-CD30 antibody treatment.

The differentiation-induced cells may also be cells derived from iPS cells into which any useful gene other than a gene for reprogramming has been introduced. Non-limiting examples of the cells include, for example, T cells derived from iPS cells into which a gene of a chimeric antigen receptor described in Themeli M. et al. Nature Biotechnology, vol. 31, no. 10, pp. 928-933, 2013. The differentiation-induced cells that are used in the method according to the present invention are derived from pluripotent stem cells.

In the present disclosure, the term "graft" refers to a construction for transplantation into a living body, and particularly means a construction for transplantation that contains cells as constituents. The graft contains at least one state of cells that are adhered to each other to constitute a certain form as a whole. A so-called suspension state of cells that are each present separately and individually is not included in the "graft" of the present disclosure. In one preferred embodiment, the graft is a construction for transplantation that does not contain constructions (e.g., a scaffold) except for cells and cell-derived substances. Examples of the graft according to the present disclosure include, but are not limited to, a sheet-shaped cell culture, multicellular spheroid, and spheroid, and the graft is preferably a sheet-shaped cell culture or multicellular spheroid, and more preferably a sheet-shaped cell culture.

In the present disclosure, the term "sheet-shaped cell culture" refers to cells that are linked to each other to form a sheet shape. In the present disclosure, the term "multicellular spheroid" refers to cells that are linked to each other to form approximately spherical shape. The cells may be linked to each other directly (including via cell components such as adhesion molecules) and/or via an intermediary substance. The intermediary substance is not particularly limited as long as it is a substance capable of at least physically (mechanically) linking cells to each other, and examples include an extracellular matrix and the like. The intermediary substance is preferably derived from a cell, in particular derived from a sheet-shaped cell culture or cells constituting the multicellular spheroid. The cells are at least linked physically (mechanically). However, the cells may be further linked functionally, for example, chemically or electrically. The sheet-shaped cell culture may be composed of one cell layer (monolayer), and may be composed of two or more cell layers (laminate (multilayer), for example, two layers, three layers, four layers, five layers, six layers, etc.). Further, the sheet-shaped cell culture may have a three-dimensional structure, in which cells do not show a clear layered structure, having a thickness that is larger than the thickness of a single cell. For example, in a vertical section of the sheet-shaped cell culture, cells may be present in the following state: cells do not aligned evenly in a horizontal direction, and a plurality of cells are arranged in a vertical direction unevenly (e.g., in mosaic).

The sheet-shaped cell culture of the present disclosure preferably does not contain a scaffold (support). The scaffold may be used in the art to stick cells on the surface and/or inside thereof and maintain physical unity of the sheet-shaped cell culture. For example, a membrane made from polyvinylidene difluoride (PVDF) is known. However, the sheet-shaped cell culture of the present disclosure can maintain the physical unity without such a scaffold. Further, the sheet-shaped cell culture of the present disclosure preferably consists only of cell-derived substances (e.g., extracellular matrix) that constitute the sheet-shaped cell culture, and does not contain other substance.

The cells may be either cells derived from a different species or cells derived from the same species. Herein, the term "cells derived from a different species" means that when a sheet-shaped cell culture is used for transplantation, the cells are derived from an organism that is different in species from the recipient of the transplantation. For example, when the recipient is a human, cells derived from a monkey or pig are the cells derived from a different species. On the other hand, the term "cells derived from the same species" refers to cells derived from an organism that is the same in species as the recipient of the transplantation. For example, when the recipient is a human, human cells are the cells derived from the same species. The cells derived from the same species include self-derived cells (also referred to as self cells or autologous cells), that is, cells derived from the recipient, and nonself-derived cells derived from the same species (also referred to as allogeneic cells). The self-derived cells do not cause graft rejection when transplanted, and thus are preferred in the present disclosure. However, the cells derived from a different species and the nonself-derived cells derived from the same species can also be used. When the cells derived from a different species or the nonself-derived cells derived from the same species are used, to prevent graft rejection, immunosuppression treatment may be required. In the present specification, cells other than the self-derived cells, that is, the cells derived from a different species and the nonself-derived cells derived from the same species may be sometimes collectively referred to as nonself-derived cells. In one embodiment of the present disclosure, cells are autologous cells or allogeneic cells. In one embodiment of the present disclosure, cells are autologous cells (including autologous cells derived from autologous iPS cells, and differentiation-induced autologous cells obtained by inducing differentiation of autologous iPS cells). In another embodiment of the present disclosure, cells are allogeneic cells (including allogeneic cells derived from allogeneic iPS cells, and differentiation-induced allogeneic cells obtained by inducing differentiation of allogeneic iPS cells).

One aspect of the present disclosure relates to a method for producing a clinical-grade graft, such as a sheet-shaped cell culture. The present inventors have found that for the manufacture of a sufficiently high-quality graft, such as a sheet-shaped cell culture, for clinical application of pluripotent stem cell-derived differentiation-induced cells, a step of removing undifferentiated cells from a cell population containing pluripotent stem cell-derived differentiation-induced cells, which are raw materials of the graft, and a step of cryopreserving the cell population are required, but the steps also cause damages to the cells contained in the cell population. The present inventors also have found that when two or more steps of these steps are carried out, a graft of sufficient quality cannot be produced according to previously known methods. Thus, the present inventors have made studies on conditions of graft production for obtaining a high-quality graft even when two or more steps of the above-described steps are carried out. As a result, the present inventors have found that a high-quality sheet-shaped cell culture can be obtained by seeding a cell population containing pluripotent stem cell-derived differentiation-induced cells at a density reaching confluence and performing sheet-forming thereof.

Accordingly, the method of the present disclosure includes the following steps:
(A) performing one single operation, or at least two different operations for removing undifferentiated cells in a cell population containing pluripotent stem cell-derived differentiation-induced cells, wherein said differentiation-induced cells are cardiomyocytes, wherein the one single operation is anti-CD30 antibody treatment, and wherein the at least two different operations comprise anti-CD30 antibody treatment; and
(B) seeding the cell population obtained in (A) on a culture substrate and performing graft-forming culture, wherein the cell population is seeded at a density reaching confluence, and wherein, in a sheet-forming incubation, a sheet culture medium used for sheet-forming incubation at day 1 contains a Rho-kinase inhibitor.

Non-limiting examples of pluripotent stem cells that can be used in the method of the present disclosure include, for example, embryonic stem cells (ES cells), induced pluripotent stem cells (iPS cells), and the like. The differentiation-induced cells that are used in the method of the present invention are cardiomyocytes.

In the method of the present disclosure, the pluripotent stem cells may be derived from any organism. Examples of such organism include, but are not limited to, humans, non-human primates, dogs, cats, pigs, horses, goats, sheep, rodents (e.g., mice, rats, hamsters, guinea pigs, etc.), rabbits, and the like Preferably, the pluripotent stem cells are human cells. In a preferred embodiment, the pluripotent stem cells are human iPS cells.

In the present disclosure, the term "operation for removing undifferentiated cells" refers to an operation for removing undifferentiated cells having tumorigenicity from a cell population containing differentiation-induced cells obtained by inducing differentiation of pluripotent stem cells. The operation applied in the method of the present invention comprises anti-CD30 antibody treatment: If one single operation is performed, this single operation is anti-CD30 antibody treatment, and if at least two different operations are performed, an anti-CD30 antibody treatment is comprised.

The method of the present invention using a specific antibody is a method of removing cells that express a marker specific to undifferentiated cells using an antibody that recognizes the undifferentiated cell marker. Examples of a marker specific to undifferentiated cells include CD30, Lin28, and the like. In the present invention, the marker specific to undifferentiated cells is CD30.

Specific examples of the method include, for example, a method using Brentuximab Vedotin. Brentuximab Vedotin is an antibody-drug complex in which an antibody that is targeted to CD30 antigen and a low molecular weight drug having microtubule inhibitory activity (monomethyl auristatin E: MMAE) are combined, and is a therapeutic agent for, for example, a recurrent and intractable CD30-positive Hodgkin's lymphoma and commercially available under the brand name of ADCETRIS. Brentuximab Vedotin can selectively act on cells that express CD30 antigen. Since CD30 antigen is highly expressed in undifferentiated cells as described above, undifferentiated cells can be removed by Brentuximab Vedotin. This method is carried out by, as a specific operation, adding Brentuximab Vedotin to a culture medium and performing incubation.

In the operation for removing undifferentiated cells, a single removal operation may be performed alone, or two or more different removal operations may be performed in combination. In a certain embodiment, in the operation for removing undifferentiated cells, a single removal operation is performed alone. The removal operation is a method of using an anti-CD30 antibody. In another embodiment, in the operation for removing undifferentiated cells, two different removal operations are performed in combination, comprising anti-CD30 antibody treatment. In still another embodiment, in the operation for removing undifferentiated cells, three or more different removal operations are performed in combination, comprising anti-CD30 antibody treatment. Examples of the combination include, for example, a combination of a method of culturing in a sugar-free medium and a method using an anti-CD30 antibody; a combination of a method using an anti-CD30 antibody and a method using heat treatment; a combination of a method of culturing in a sugar-free medium, a method using an anti-CD30 antibody, and a method using heat treatment; and the like. By combining a plurality of these operations for removing undifferentiated cells, a synergistic effect of removing undifferentiated cells can be achieved. Specific examples of the combination of operations for removing undifferentiated cells include the following: at first, heat treating iPS cell-derived differentiation-induced cells by, for example, a method described in WO 2017/038562, then culturing in a sugar-free medium by, for example, a method described in WO 2007/088874, and thereafter performing an anti-CD30 antibody-conjugated drug treatment method described in WO 2016/072519.

In one preferred embodiment, the operations for removing undifferentiated cells are performed by using at least two methods, wherein the at least two different methods comprise anti-CD30 antibody treatment.

The cell population containing pluripotent stem cell-derived differentiation-induced cells may be subjected to an operation for removing undifferentiated cells, then optionally seeded and adhesively cultured on a culture substrate (preferably, on a planar culture substrate), and thereafter subjected to a step of collecting the cultured cells. The adhesively culturing step may be performed before a cryopreservation step described below, or performed after cryopreservation and thawing. By performing the adhesively culturing step, dead cells can be efficiently removed. Thus, in the following graft formation, a high-quality graft can be formed at a high probability.

In the adhesively culturing step, culturing conditions or the like may be those based on conditions for performing usual adhesive culture. For example, the culture may be performed using a commercially available culture vessel for adhesive culture under conditions at 37°C and 5% CO₂. Seeding densities of cells may be any density as long as cell-cell adhesion and/or adhesion between a cell and a culture substrate are not prevented. For example, the density may be a density of subconfluence, or may be a density reaching confluence or higher. In the method of the present invention, the cell population is seeded at a density reaching confluence. Culture time is not particularly limited as long as the time may allow cell-cell adhesion and/or adhesion between a cell and a culture substrate to be produced. Specifically, the time may be, for example, about 2 to 168 hours, about 2 to 144 hours, about 2 to 120 hours, about 2 to 96 hours, about 2 to 72 hours, about 2 to 48 hours, about 2 to 24 hours, about 2 to 12 hours, about 2 to 6 hours, and about 2 to 4 hours.

Alternatively, the above-described operation for removing undifferentiated cells may be performed in the adhesively culturing step. For example, treatment with the specific anti-CD30 antibody may be performed during adhesive culture in the adhesively culturing step.

For collecting the adhesively cultured cells, methods publicly known in the art may be used. Specific examples include, for example, the following: treating adhesively cultured cells with a protease such as trypsin or TrypLE TM Select, and collecting the resulting dissociated cells. In addition, optionally, the collected cells may be washed.

The adhesively culturing step can be performed in combination with a plurality of operations for removing undifferentiated cells. When the adhesively culturing step is performed in combination with a plurality of operations for removing undifferentiated cells, the adhesively culturing step may be performed before the operations for removing undifferentiated cells, after the operations for removing undifferentiated cells, or between each of the operations for removing undifferentiated cells. That is, the plurality of operations for removing undifferentiated cells and the adhesively culturing step may be performed in any combination. As specific examples, but are not limited to, when the plurality of operations for removing undifferentiated cells are denoted by operation A, operation B, and operation C, and the adhesively culturing step is denoted by X, the order of the operations may be A → B → X, A → C → X, B → C → X, A → B → C → X, A → C → B → X, B → A → C → X, B → C → A → X, C → B → A → X, C → A → B → X, X → A → B, X → A → C, X → B → C, X → A → B → C, X → A → C → B, X → B → A → C, X → B → C → A, X → C → B → A, X → C → A→ B, A → X → B, A → X → C, B → X → C, A → X → B → C, A → X C → B, B → X → A → C, B → X → C → A, C → X → A → B, C → X B → A, A → B → X → C, A → C → X → B, B → A → X → C, B → C X → A, C → A → X → B, or C → B → X → A, and the like.

The cell population containing pluripotent stem cell-derived differentiation-induced cells may optionally, after performing the operation for removing undifferentiated cells, be cryopreserved. The cryopreservation may include a step of freezing cells (a cell population), and a step of thawing the frozen cells. The freezing of cells can be performed by any method known in the art. Examples of the method include, but are not limited to, the following: exposing cells in a vessel to, for example, freezing means, such as a freezer, a deep freezer, a low-temperature medium (e.g., liquid nitrogen) or the like. The temperature of the freezing means is not particularly limited as long as a part, preferably all of the cell population in the vessel can be frozen at the temperature. Typically, the temperature is about 0°C or less, preferably about -20°C or less, more preferably about -40°C or less, and still more preferably about -80°C or less. The cooling rate of the freezing operation is not particularly limited as long as survival rates or functions of the cells after freezing and thawing are not largely reduced. Typically, the cooling rate is such that cooling from 4°C until about -80°C requires about 1 hour to about 5 hours, preferably about 2 hours to about 4 hours, particularly preferably about 3 hours. Specifically, the cooling can be performed at a rate of about 0.46°C/min. The cooling rate can be achieved as follows: exposing a vessel containing cells directly to freezing means that is set at a desired temperature, or exposing a vessel containing cells that are accommodated in a freezing treatment vessel to the freezing means. The freezing treatment vessel may have a function to perform control such that the temperature dropping speed in the vessel becomes a predetermined speed. As the freezing treatment vessel, any vessels known in the art, such as BICELL (registered trademark) (Nihon Freezer Co., Ltd.), a program freezer, and the like can be used.

The freezing operation may be performed while cells are still soaked with a culture solution or a physiological buffer. However, the freezing operation may be performed after the cells are subjected to a treatment of adding a cryoprotective agent for protecting the cells from the freezing and thawing operations to the culture solution, replacing the culture solution with a cryopreservation liquid containing a cryoprotective agent, or the like. Thus, the production method of the present disclosure including a freezing step may further include a step of adding a cryoprotective agent to the culture solution, or a step of replacing the culture solution with a cryopreservation liquid. When the culture solution is replaced with a cryopreservation liquid, as long as an effective concentration of the cryoprotective agent is contained in a liquid with which the cells are soaked during freezing, the cryopreservation liquid may be added after removing substantially all of the culture solution, or the cryopreservation liquid may be added while a part of the culture solution still remains. Herein, the term "effective concentration" refers to a concentration at which the cryoprotective agent shows, without showing toxicity, a freeze-protective effect as compared to a case in which the cryoprotective agent is not used. Examples of the freeze-protective effect include an inhibitory effect on reduction in survival rates, vitalities, functions, or the like of cells after freezing and thawing. Such a concentration is known by those skilled in the art, or can be suitably determined by a routine experiment.

The cryoprotective agent is not particularly limited as long as it shows a cryoprotective action on cells. Examples include dimethyl sulfoxide (DMSO), glycerol, ethylene glycol, propylene glycol, sericin, propanediol, dextran, polyvinylpyrrolidone, polyvinyl alcohol, hydroxyethyl starch, chondroitin sulfate, polyethylene glycol, formamide, acetamide, adonitol, perseitol, raffinose, lactose, trehalose, sucrose, mannitol, and the like. The cryoprotective agents may be used alone or in combination of two or three or more.

The concentration of the cryoprotective agent added to the culture solution, or the concentration of the cryoprotective agent in the cryopreservation liquid is not particularly limited as long as the concentration is within the above-defined effective concentration. Typically, the concentration is about 2% to about 20% (v/v) with respect to the total amount of the culture solution or the cryopreservation liquid. However, even without the concentration range, an alternative concentration that is known or experimentally determined about each cryoprotective agent can be used, and the concentration also falls within the scope of the present disclosure.

The step of thawing frozen cells can be performed by any cell thawing protocols known in the art. Typically, the step can be performed as follows: for example, exposing frozen cells to thawing means, such as a solid, liquid, or gaseous medium (e.g., water), a water bath, an incubator, a thermostat oven, or the like having a temperature that is higher than the freezing temperature, or soaking frozen cells with a medium (e.g., culture solution) having a temperature that is higher than the freezing temperature. But the method of thawing frozen cells is not limited thereto. Thawing means or temperature of the soaking medium is not particularly limited as long as cells can be thawed within a desired time. Typically, the temperature is about 4°C to about 50°C, preferably about 30°C to about 40°C, and more preferably about 36°C to about 38°C. Thawing time is not particularly limited as long as survival rates or functions of the cells after thawing are not largely reduced. Typically, the thawing time is within about 2 minutes. In particular, when the thawing time is within about 20 seconds, reduction in the survival rates can be significantly prevented. The thawing time can be regulated by changing the temperature of the thawing means or soaking medium, the volume or composition of the culture solution or cryopreservation liquid for freezing, or the like. The frozen cells include cells frozen by any method, and non-limiting examples of the cells include cells frozen by the above-described step of freezing cells. In one embodiment, the frozen cells are cells frozen in the presence of a cryoprotective agent. In one embodiment, the frozen cells are cells for use in the production method of the present disclosure.

The method of the present disclosure may include, after a step of thawing the frozen cells and before a step of forming a graft, a step of washing cells. The washing of cells may be performed by any method known in the art. Typically, the washing can be performed as follows: for example, suspending cells in a washing solution (e.g., a culture solution (e.g., culture medium or the like), a physiological buffer (e.g., PBS, HBSS), or the like containing or not containing serum or a component of serum (serum albumin or the like), or the like), centrifuging the suspension, discarding the resulting supernatant, and collecting precipitated cells. But the washing is not limited thereto. In the step of washing cells, the cycle of suspending, centrifuging, and collecting may be performed once, or a plurality of times (e.g., twice, 3 times, 4 times, 5 times, or the like). In one embodiment of the present disclosure, the step of washing cells is performed immediately after a step of thawing frozen cells.

The method of the present disclosure includes a step of thawing a frozen cell population, then seeding the cell population on a culture substrate, and forming a graft. Depending on shapes of the graft, culture substrate for seeding, density of cells, or the like may vary. For example, when the graft is a sheet, the step include seeding cells on a planar cell-adhesive culture substrate, and when the graft is a sphere, the step include seeding cells on a non-cell-adhesive substrate, or the like. Those skilled in the art can suitably select an optimum condition. The present invention will be described in detail below using a case, as an example, in which the graft is a sheet-shaped cell culture. When the graft is a sheet-shaped cell culture, the method of the present disclosure include a step of seeding the above-described cell population on a culture substrate at a density reaching confluence and performing sheet-forming thereof. The sheet-forming of cells can be performed by any method and conditions known in the art. It is thought that the sheet-forming is achieved by adhesion of cells to each other via an intercellular adhesion mechanism such as an adhesion molecule or an extracellular matrix. Thus, the step of sheet-forming of the seeded cells can be achieved by, for example, culturing cells under conditions that allow the cells to form intercellular adhesion. Such conditions may be any conditions as long as the intercellular adhesion can be formed. Generally, under conditions about the same as usual cell culturing conditions, the intercellular adhesion can be formed. Examples of culturing under such conditions include culturing under conditions at 37°C and 5% CO₂. In addition, culture can be performed under normal pressure (atmospheric pressure). Those skilled in the art can select optimum conditions depending on cells to be seeded. In the present specification, culturing seeded cells and forming a graft is designated as "graft-forming culture". In particular, when the graft is a sheet-shaped cell culture, the graft-forming culture may be sometimes referred to as "sheet-forming incubation". Non-limiting examples of the sheet-forming incubation are described in Patent Literature 1, JP 2010-081829 A, JP 2010-226991 A, JP 2011-110368 A, JP 2011-172925 A, WO 2014/185517, and the like.

The surface of the culture substrate may be covered with materials in which physical properties change in response to a stimulation, such as temperature or light. As the materials, publicly known materials may be used, and examples of the publicly-known material include, but are not limited to, temperature responsive materials including a homopolymer or a copolymer of (meth)acrylamide compounds, N-alkyl-substituted (meth)acrylamide derivatives (e.g., N-ethyl acrylamide, N-n-propyl acrylamide, N-n-propyl methacrylamide, N-isopropyl acrylamide, N-isopropyl methacrylamide, N-cyclopropyl acrylamide, N-cyclopropyl methacrylamide, N-ethoxyethyl acrylamide, N-ethoxyethyl methacrylamide, N-tetrahydrofurfuryl acrylamide, N-tetrahydrofurfuryl methacrylamide, and the like), N,N-dialkyl-substituted (meth)acrylamide derivatives (e.g., N,N-dimethyl(meth)acrylamide, N,N-ethylmethyl acrylamide, N,N-diethyl acrylamide, and the like), (meth)acrylamide derivatives having a cyclic group (e.g., 1-(1-oxo-2-propenyl)-pyrolidine, 1-(1-oxo-2-propenyl)-piperidine, 4-(1-oxo-2-propenyl)-morpholine, 1-(1-oxo-2-methyl-2-propenyl)-pyrolidine, 1-(1-oxo-2-methyl-2-propenyl)-piperidine, 4-(1-oxo-2-methyl-2-propenyl)-morpholine, and the like), or vinyl ether derivatives (e.g., methyl vinyl ether); and photoresponsive materials such as a light-absorbing polymer having an azobenzene group, a copolymer of a vinyl derivative of triphenylmethane leucohydroxide and an acrylamide-based monomer, N-isopropylacrylamide gel containing spirobenzopyran, and the like (see, for example, JP 2-211865 A and JP 2003-33177 A). By applying a predetermined stimulus to these materials, the properties such as hydrophilicity or hydrophobicity can be changed, and detachment of a cell culture sticking on the materials can be facilitated. Culture dishes covered with a temperature responsive material are commercially available (e.g., UpCell (registered trademark) from CellSeed Inc.), and they can be used for the production method of the present disclosure.

The above-described culture substrate may have various shapes. Preferably, the culture substrate is planar. The area of the culture substrate is not particularly limited, and may be about 1 cm² to about 200 cm², about 2 cm² to about 100 cm², about 3 cm² to about 50 cm², or the like.

The culture substrate may be coated (covered or coated) with other coating agents such as serum and/or cell-adhesive components (sometimes collectively referred to as "coating components"). By using a culture substrate coated with the coating components, a further high-density sheet-shaped cell culture can be formed. The term "coated with coating components" refers to a state in which the coating components are stuck on the surface of the culture substrate. Such a state can be achieved by, for example, treating the culture substrate with coating components, but the method of coating is not limited thereto. The treatment with coating components include bringing the coating components into contact with the culture substrate, and, if necessary, incubating for a predetermined time. The temperature of the incubation is not particularly limited, and may be, for example, 4°C to 37°C. As the serum, heterologous serum and homologous serum can be used. The heterologous serum refers to, when the cell culture is used for transplantation, serum derived from a species that is different from the recipient. For example, when the recipient is a human, serum derived from cattle or horses, such as fetal bovine serum (FBS or FCS), calf serum (CS), horse serum (HS), or the like, falls within the definition of "heterologous serum". On the other hand, the term "homologous serum" refers to serum derived from a species that is the same as the recipient. For example, the recipient is a human, human serum falls within the definition of "homologous serum". The homologous serum includes autologous serum (also referred to as self-serum), that is, serum derived from the recipient, and nonautologous serum derived from the same species, which is derived from other individuals of the same species In the present specification, serum other than autologous serum, that is, heterologous serum and nonautologous serum derived from the same species may be sometimes collectively referred to as nonself serum. Examples of the above-described other coating agent include cell-adhesive components such as an extracellular matrix, a cell adhesion factor, and the like. Examples of the cell-adhesive component include, but are not limited to, extracellular matrices such as collagen, fibronectin, laminin, vitronectin, proteoglycan, glycosaminoglycan, and the like; and cell adhesion factors such as the cadherin family, the selectin family, the integrin family, and the like. The cell-adhesive components of the present disclosure also include modification products of the foregoing (e.g., a polypeptide containing a functional domain). Examples of the modification products of the foregoing include laminin 511 and laminin 211 (modification products of laminin), VTN-N (a modification product of vitronectin), RetroNectin (registered trademark) (a modification product of fibronectin), and the like.

The coating components for coating a culture substrate may be commercially available, or can be prepared from a biological sample obtained from a desired organism by a conventional method. Specifically, for example, examples of a method for preparing serum include a method of coagulating drawn blood by allowing to stand for about 20 minutes to about 60 minutes at room temperature, centrifuging the coagulation by about 1000 × g to about 1200 × g, and collecting the supernatant, or the like.

For incubating on a culture substrate, the coating components may be used as an undiluted solution, or as a diluted solution. The dilution may be performed by using any medium, and examples of the medium include, but are not limited to, water, physiological saline, various buffers (e.g., PBS, HBS, or the like), various liquid culture media (e.g., DMEM, MEM, F12, DME, RPMI1640, MCDB (MCDB 102, 104, 107, 120, 131, 153, 199, or the like), L15, SkBM, RITC80-7, DMEM/F12, or the like), and the like. The concentration of the diluted solution is not particularly limited as long as the coating components can stick on a culture substrate, and examples include about 0.5% to about 100% (v/v), preferably about 1% to about 60% (v/v), and more preferably about 5% to about 40% (v/v).

In the present disclosure, the term "density reaching confluence" is a density at which cells are expected to cover the entire adhesion surface of the culture vessel when the cells are seeded. For example, the density reaching confluence is a density at which, when cells are seeded, the cells are expected to come into contact with each other, a density at which contact inhibition occurs, or a density at which cell growth is substantially stopped by contact inhibition. The density reaching confluence can be calculated by those skilled in the art from the size of desired cells and the area of the adhesion surface of the culture vessel. Therefore, those skilled in the art may also suitably determine the optimum seeding density. The upper limit of the seeding density is not particularly limited, but if the density is excessively high, many cells die, leading to inefficiency. In one embodiment of the present invention, examples of the seeding density include about 0.4×10⁶ cells/cm² to about 1.0×10⁷ cells/cm², about 0.4×10⁶ cells/cm² to about 5.0×10⁶ cells/cm², about 0.4×10⁶ cells/cm² to about 3.0×10⁶ cells/cm², about 0.5×10⁶ cells/cm² to about 1.0×10⁷ cells/cm², about 0.5×10⁶ cells/cm² to about 5.0×10⁶ cells/cm², about 0.5×10⁶ cells/cm² to about 3.0×10⁶ cells/cm², about 1.0×10⁶ cells/cm² to about 1.0×10⁷ cells/cm², about 1.0×10⁶ cells/cm² to about 5.0×10⁶ cells/cm², about 1.0×10⁶ cells/cm² to about 3.0×10⁶ cells/cm², about 1.5×10⁶ cells/cm² to about 1.0×10⁷ cells/cm², about 1.5×10⁶ cells/cm² to about 5.0×10⁶ cells/cm², about 1.5×10⁶ cells/cm² to about 3.0×10⁶ cells/cm², about 2.0×10⁶ cells/cm² to about 1.0×10⁷ cells/cm², about 2.0×10⁶ cells/cm² to about 5.0×10⁶ cells/cm², about 2.0×10⁶ cells/cm² to about 3.0×10⁶ cells/cm². In one preferred embodiment, the seeding density is about 0.40 to 2.33×10⁶ cells/cm², more preferably about 1.05×10⁶ cells/cm² to about 2.33×10⁶ cells/cm², and still more preferably about 1.76×10⁶ cells/cm² to about 2.33×10⁶ cells/cm².

The time of sheet-forming incubation may vary depending on types of cells to be seeded and cell densities. For example, when cardiomyocytes are prepared from iPS cells to perform sheet-forming thereof, the sheet-forming has been performed by seeding at a density of, for example, about 2.1×10⁵ cells/cm² or the like and culturing for 4 days or more. On the other hand, in the method of the present disclosure, by using a density reaching confluence as a seeding density, that is, by seeding at a density that is higher than that in conventional methods, the time for the sheet-forming incubation can be reduced. For seeding at a high density to perform sheet-forming incubation, since the formed sheet-shaped cell culture becomes susceptible to detachment from the culture substrate after the sheet has been formed, it is not preferred to perform the sheet-forming incubation for a long period of time. Thus, in one embodiment of the present disclosure, the sheet-forming incubation is performed for 2 to 4 days, and more preferably 2 to 3 days.

The medium used for the sheet-forming (sometimes also referred to as a sheet culture medium) is not particularly limited as long as sheet-forming of cells becomes possible. For example, physiological saline, various physiological buffers (e.g., PBS, HBSS, or the like), media based on various basal media for cell culture may be used. Examples of the basal media include, but are not limited to, DMEM, MEM, F12, DME, RPMI1640, MCDB (MCDB 102, 104, 107, 120, 131, 153, 199, or the like), L15, SkBM, RITC80-7, DMEM/F12, and the like. Most of the basal media are commercially available, and the formulations of the media are publicly known. The basal media may be used in a standard formulation (e.g., in a state as it is commercially distributed), or the formulation may be suitably changed depending on types of cells and cell conditions. Thus, the basal media used for the present invention are not limited to those of publicly known formulations, but include those in which one or two or more components are added, removed, increased, or decreased. The sheet culture medium may contain additives such as serum (e.g., cattle serum such as fetal bovine serum, horse serum, human serum, or the like), various factors (e.g., FGF, EGF, VEGF, HGF, or the like). When FBS is used, 20% FBS (10% or more and less than 25%, more preferably 15% or more and less than 25%) is preferred.

The sheet culture medium may be suitably replaced during sheet-forming incubation. The formulation of the medium may be changed depending on the progress of sheet-forming. The present inventors have newly found that when a sheet culture medium supplemented with a Rho-kinase (ROCK) inhibitor is used as medium at day 1 of sheet-forming incubation, a sheet-shaped cell culture is efficiently formed. Thus, in the method of the present disclosure, the sheet culture medium for use in sheet-forming incubation at day 1 contains a Rho-kinase inhibitor. In the embodiment, the sheet culture medium at day 2 or later may contain or not contain a Rho-kinase inhibitor, and preferably does not contain a Rho-kinase inhibitor.

In addition, the above-described operation for removing undifferentiated cells may be performed during sheet-forming incubation. For example, during the sheet-forming incubation, treatment with heat or a specific antibody may be performed, or a part of the sheet-forming incubation may be performed in a sugar-free medium.

In addition, the present inventors have paid attention to the finding that when dead cells are present in a cell population that are seeded on a culture substrate, the dead cells tend to be adhered to each other to form aggregates. Then, the present inventors have found that the aggregates of dead cells have an influence on breakage and perforation of a sheet-shaped cell culture at sheet-forming. In addition, the present inventors have newly found that by removing the aggregates before seeding, the risk of breakage of the sheet-shaped cell culture can be reduced. Thus, in another aspect, the present disclosure relates to a method for producing a sheet-shaped cell culture including a step of performing a treatment for removing dead cells before seeding a cell population containing sheet-forming cells (e.g., pluripotent stem cell-derived differentiation-induced cells, or the like) on a culture substrate.

In the present disclosure, for removing dead cells, any means for removing dead cells known in the art can be used. Examples of the means for removing dead cells include, but are not limited to, a method of performing filtering treatment using a filter such as a cell strainer, a separation method using a cell sorter, a separation method using magnetic beads, a method using density gradient centrifugation, a method of dissociating aggregation of living cells and dead cells using an enzyme such as DNase, and the like. From the viewpoint of convenience or the like, a method of performing filtering treatment is preferred.

When dead cells or aggregates formed by aggregation of the dead cells are present together in a cell population that is to be seeded, the thicknesses of a sheet-shaped cell culture formed therefrom may become uneven, or a part of the sheet may not be formed, leading to an increased risk of causing breakage or perforation of the sheet-shaped cell culture. Thus, when these cells are removed, living cells are evenly distributed on a culture substrate, and the thickness of a sheet-shaped cell culture formed therefrom becomes even, and therefore the risk of breakage of the sheet-shaped cell culture can be reduced.

In the present disclosure, the term "filtering treatment" refers to filtering a cell population using a filter having a predetermined pore size, and separating and removing materials having particle sizes that are as large as or larger than the pore size. For example, when a cell population is filtered by using a filter, such as a commercially available cell strainer, having a predetermined pore size of, for example, 500 µm, 200 um, or 100 µm, aggregates formed by aggregation of dead cells are removed. Thus, a cell population that has passed through the filter is clear of dead cells.

The pore size of a filter is not particularly limited as long as the pore size allows cell aggregates to be separated from living cells. Thus, it is sufficient that the upper limit of the pore size is smaller than the aggregates of dead cells, for example, 500 um or less, 200 um or less, 150 um or less, 100 um or less, 85 um or less, 70 um or less, 40 um or less, or the like. On the other hand, it is sufficient that the lower limit of the pore size is larger than a size that allows living cells to pass through, for example, 10 um or more, 15 um or more, 20 um or more, 40 um or more, or the like. Alternatively, two or more filters having different pore sizes may be used in combination for filtering.

It is known that when cell membranes of dead cells in a cell population have been broken and nucleic acids (e.g., DNA) in the cells are leaked, living cells tend to be trapped therein to form aggregates (cell aggregates). Such aggregates may have detrimental effects including impairing uniformity in formation of a graft (in particular, sheet-shaped cell culture or the like), and may cause perforation or the like. By passing through a filter having the above-described pore size, such aggregates are removed from a cell population. As a result, although, for example, 6 or more aggregates (cell aggregates) caused by the dead cells are present per field of view (e.g., area of the field of view under 10-fold magnification is about 3 cm²) before the filtering treatment, the number of aggregates (cell aggregates) caused by dead cells counted under the same conditions are reduced after the filtering treatment, for example, to 5 or less to zero, 4 or less to zero, 3 or less to zero, 2 or less to zero, or 1 or less to zero. More favorable grafts can be obtained as the number of such aggregates decreases. From the viewpoint of removing cell aggregates, the pore size of a filter is preferably, as described above, 40 um to 100 µm.

The method of the present aspect is particularly effective when a sheet-shaped cell culture is formed using limited resources, for example, when a sheet is formed using cells differentiated from pluripotent stem cells by inducing the differentiation. Thus, in the method of the present invention, sheet-forming cells are cardiomyocytes differentiated from pluripotent stem cells (e.g., human iPS cells or the like) by inducing the differentiation. In one embodiment, the formed sheet of cardiomyocytes may contain, in addition to cardiomyocytes, vascular endothelial cells, wall cells, and fibroblast cells. Percentages of the cells contained as constituents in the sheet-shaped cell culture of the present disclosure may be, for example, as follows: cardiomyocytes: about 30 to 70%, vascular endothelial cells: 0.1% to about 20%, and wall cells: about 1% to about 40%.

In the method of the present aspect, sheet-forming incubation may be performed by any method known in the art. However, in particular, sheet-forming incubation without cell proliferation is remarkably effective in sheet formation. Examples of the sheet-forming incubation without cell proliferation include sheet-forming incubation of low-proliferative cells such as cardiomyocytes, sheet-forming incubation when a cell population is seeded at a density reaching confluence, and the like. In one embodiment, sheet-forming incubation without cell proliferation is performed, from a step of thawing frozen cells to a step of performing sheet-forming incubation via a step of removing dead cells, under conditions in which cell proliferation does not occur.

As described above, in clinical application of a sheet-shaped cell culture, the sheet-forming cells are preferably capable of being cryopreserved. However, for example, when pluripotent stem cell-derived differentiation-induced cells or the like are cryopreserved and thereafter the frozen cells are thawed, a certain amount of the cells become dead cells. Thus, the method of the present aspect can be suitably used, in particular, when a cell population containing sheet-forming cells are once cryopreserved and thereafter thawed and collected.

One particularly preferred embodiment of the method for producing a sheet-shaped cell culture of the present disclosure includes the following steps:
(a) performing one single operation, or at least two different operations for removing undifferentiated cells in a cell population containing pluripotent stem cell-derived cardiomyocytes, wherein the one single operation is anti-CD30 antibody treatment, and wherein the at least two different operations comprise anti-CD30 antibody treatment;
(b) cryopreserving the cell population obtained in (a);
(c) thawing the cell population cryopreserved in (b);
(d) performing filtering treatment of the cell population thawed in (c); and
(e) seeding the cell population subjected to the filtering treatment in (d) on culture substrate at a density reaching confluence and performing sheet-forming incubation.

In the embodiment, each of the steps of (a) to (e) is as described in detail above. In addition, the method may include other steps, such as:
(f) a step of detaching the sheet-shaped cell culture formed in (e) from the culture substrate;
(g) a step of seeding and adhesively culturing the cell population on an adhesive culture substrate, and thereafter collecting the cell population; and the like.

Further disclosed herein, however not being part of the present invention is a method for producing a graft including a step of adhesively culturing. The method for producing a graft of the present aspect include the following steps:
(a) a step of dispersing embryonic bodies to obtain a cell population;
(b) a step of seeding and adhesively culturing the cell population obtained in (a) on a culture substrate, and thereafter collecting the cell population; and
(c) seeding the cell population obtained in (b) on a culture substrate and performing graft-forming culture.

In step (a), the embryonic bodies obtained by inducing differentiation from pluripotent stem cells such as iPS cells are dispersed by treating with a protease such as trypsin or TrypLE^{™} Select to obtain a cell population. The dispersion of embryonic bodies is publicly known in the art, and can be performed according to, for example, methods described in Miki et al., Cell Stem Cell 16, 699-711, June 4, 2015, WO 2014/185358, and the like.

In step (b), the cell population obtained by dispersing embryonic bodies is subjected to adhesive culture, and thereafter the cultured cells are collected. The adhesively culturing step and the collecting step are as described in detail above. As described above, by the adhesive culture, dead cells can be effectively removed, and the cell population obtained by collection shows high viability.

The cell population obtained in step (b) may be subjected to graft-forming culture as it is. However, after step (b), a cryopreservation step and a thawing step may be optionally included. The cryopreservation step and the thawing step are as described in detail above.

In step (c), by seeding the cell population on a culture substrate to form a graft. The step of forming a graft is also as described in detail above.

As described above, the graft obtained by the method of the present disclosure can be preferably used for, in particular, clinical use, that is, used in a method of treatment of diseases. Thus, the graft contains any graft-forming cells (e.g., pluripotent stem cell-derived differentiation-induced cells) which are assumed to be applied to organs and apparatuses of a subject in need thereof. Non-limiting examples of the graft-forming cells include, for example, cells applied to a heart, blood, a blood vessel, a lung, a liver, a pancreas, a kidney, a large intestine, a small intestine, a spinal cord, a central nervous system, a bone, an eye, or skin. The graft is applied to a subject for use in treating a disease. A cell culture or a composition for use in a method of treating a disease can contain a graft as an active ingredient prepared by a method of the present disclosure. Examples of the disease include, but are not limited to, heart disease, blood disease, vascular disease, lung disease, liver disease, pancreas disease, kidney disease, large intestine disease, small intestine disease, spinal cord disease, central nervous system disease, bone disease, eye disease, or skin disease. When the graft-forming cells are cardiomyocytes, examples include myocardial infarction (including chronic heart failure associated with myocardial infarction), dilated cardiomyopathy, ischemic cardiomyopathy, heart disease (e.g., heart failure, in particular, chronic heart failure) accompanied by systolic dysfunction (e.g., left ventricular systolic dysfunction). The disease may be a disease in which a graft of the present disclosure is useful for treating the disease.

Another aspect relates to an effective amount of a graft produced by the method of the present disclosure for use in a method of treating a disease of a subject in need thereof. The diseases to be treated are as described above.

In the present disclosure, the term "treatment" is intended to encompass all kinds of medically acceptable prophylactic and/or therapeutic interventions aimed at curing, temporary remission, or prevention of diseases. For example, the term "treatment" includes medically acceptable interventions for a variety of purposes, including delaying or halting the progression of a disease associated with tissue abnormalities, regression or disappearance of a lesion, prevention of the onset of the disease, or prevention of recurrence.

In the treatment method, a component that enhances the viability, engraftment, and/or function of a graft, other active ingredients useful for treating a targeted disease, or the like can be used in combination with the graft and the like of the present disclosure.

The treatment method may further include a step of producing the graft of the present disclosure according to the foregoing production method of the present disclosure. The treatment method may further include, before the step of producing the graft, a step of collecting cells (e.g., skin cells, hemocytes, or the like when using iPS cells) for producing the graft from a subject, or tissue (e.g., skin tissue, blood, or the like when using iPS cells) as a source of the cells. In one embodiment, the subject for collecting the cells or tissue as a source of the cells is an individual that is the same as the subject that receives administration of the cell culture, the composition, the graft, or the like. In another embodiment, the subject for collecting the cells or tissue as a source of the cells is a different individual of the same species as the subject that receives administration of the cell culture, the composition, the graft, or the like. In another embodiment, the subject for collecting the cells or tissue as a source of the cells is an individual of a different species from the subject that receives administration of the graft or the like.

In the present disclosure, an effective amount is, for example, an amount (for example, size, weight, number of sheets, or the like of sheet-shaped cell culture) that is capable of suppressing the onset or recurrence of a disease, reducing symptoms, or delaying or stopping the progression, and preferably, the effective amount is an amount that is capable of preventing the onset and recurrence of the disease or curing the disease. Also, an amount that does not cause detrimental effects that cancel the benefits of administration is preferred. Such amount may be suitably determined by, for example, performing a test using experimental animals or disease model animals, such as mice, rats, dogs or pigs. Methods for performing the test are well known to those skilled in the art. In addition, the size of the tissue lesion to be treated may be an important indicator for determining the effective amount.

Examples of methods of administration include intravenous administration, intramuscular administration, intraosseous administration, intrathecal administration, direct application to tissues, and the like. Although the frequency of administration is typically once per treatment, multiple administrations may also be possible if the desired effect cannot be achieved. When applied to tissue, the cell culture, composition, graft and the like of the present invention may be fixed to the target tissue by locking means such as sutures or staples.

### Examples

The present invention will be described in more detail with reference to the following examples, which illustrate specific examples of the present invention, but the present invention does not limited thereto.

In the following examples, human iPS cells for clinical use established by Center for iPS Cell Research and Application, Kyoto University (CiRA) were used as pluripotent stem cells, and was maintained by a feeder free method with reference to M. Nakagawa et al., Scientific Reports, 4:3594 (2014). Embryonic bodies were obtained by inducing differentiation into cardiomyocytes with reference to the descriptions in Miki et al., Cell Stem Cell 16, 699-711, June 4, 2015, WO 2014/185358, and WO 2017/038562. Specifically, human iPS cells maintained and cultured in a feeder-cell-free culture solution were cultured on EZ Sphere (Asahi Glass Co., Ltd.) in StemFit AK03 medium (AJINOMOTO CO., INC.) containing 10 µM of Y27632 (Wako Pure Chemical Industries, Ltd.) for 1 day to obtain embryonic bodies. The obtained embryonic bodies were cultured in a culture solution containing activin A, bone morphogenetic protein (BMP) 4, and basic fibroblast growth factor (bFGF), further cultured in a culture solution containing a Wnt inhibitor (IWP-3), a BMP4 inhibitor (Dorsomorphin), and a TGFβ inhibitor (SB431542), and thereafter cultured in a culture solution containing VEGF and bFGF to obtain a cell population. The content of cardiomyocytes in the obtained cell population was 50% to 90%.

### Example 1. Examination of conditions for sheet-forming incubation

When a sheet-shaped cell culture for clinical use was formed by using the above-obtained cell population containing cardiomyocytes differentiated from human iPS cells by inducing the differentiation, it tended to become more difficult to form the sheet-shaped cell culture as more steps causing reduction in cell viability, such as removing undifferentiated cells, and freezing and thawing a cell population, were added. Thus, by performing sheet-forming incubation under various conditions with different numbers of culture days, different sheet culture media, and the like, conditions of sheet-forming incubation for suitably forming a sheet-shaped cell culture even in a cell population in which cell viability was considered to be decreased.

As Comparative Example, a method described in Example 2 of WO 2017/038562 was used, and a comparison was performed with Production Examples 1 and 2. The sheet-forming method in each example will be briefly described below.

Comparative Example: Cardiomyocytes were differentiated from human iPS cells by inducing the differentiation according to the above-described method, and thereafter undifferentiated cells were removed by heat treatment at 42°C and sheet-forming incubation was performed.

Production Example 1: Cardiomyocytes were differentiated from human iPS cells by inducing the differentiation according to the above-described method, and thereafter undifferentiated cells were removed by using anti-CD30 antibody-conjugated drug treatment described in WO 2016/072519 to obtain a cell population. Then, the cell population was cooled to -80°C at a cooling rate of - 1°C/min using a program freezer, then cryopreserved in liquid nitrogen, thereafter thawed, and subjected to sheet-forming incubation. The culture substrate used had been prepared on the day before cell seeding by adding FBS-containing DMEM to a temperature responsive culture dish, and performing coating at 37°C. In the sheet-forming incubation, cells were seeded, thereafter cultured under conditions at 37°C and 5% CO₂, and the culture medium was replaced daily. After the culture was completed, the cells were detached to form a sheet by decreasing the temperature. Only at day 1 of the sheet-forming incubation, a Rho-kinase inhibitor Y27632 was added to the sheet culture medium. After day 2 of the sheet-forming incubation, the Rho-kinase inhibitor Y27632 was removed from the sheet culture medium (the expression "only at day 1 of the sheet-forming incubation, a Rho-kinase inhibitor Y27632 was added to the sheet culture medium" means that after day 2 of the sheet-forming incubation, the sheet culture medium did not contain the Rho-kinase inhibitor Y27632. The same applies hereinafter.).

Production Example 2: Cardiomyocytes were differentiated from human iPS cells by inducing the differentiation according to the above-described method, and thereafter undifferentiated cells were removed by using heat treatment described in WO 2017/038562, a sugar-free medium culture method described in WO 2007/088874, and anti-CD30 antibody-conjugated drug treatment described in WO 2016/072519 in this order to obtain a cell population. Then, the cell population was cooled to -80°C at a cooling rate of -1°C/min using a program freezer, then cryopreserved in liquid nitrogen, thereafter thawed, and subjected to sheet-forming incubation. The culture substrate used had been prepared on the day before cell seeding by adding FBS-containing DMEM to a temperature responsive culture dish, and performing coating at 37°C. In the sheet-forming incubation, cells were seeded, thereafter cultured under conditions at 37°C and 5% CO₂, and the culture medium was replaced daily. After the culture was completed, the cells were detached to form a sheet by decreasing the temperature. Only at day 1 of the sheet-forming incubation, a Rho-kinase inhibitor Y27632 was added to the sheet culture medium. The steps of the sheet-forming incubation are shown below.

**[Table 1]**

| | Comparative Example | Production Example 1 | Production Example 2 |
|---|---|---|---|
| Heat treatment | ○ | × | ○ |
| Sugar-free culture | × | × | ○ |
| Anti-CD30 antibody treatment | × | ○ | ○ |
| Freezing and thawing | × | ○ | ○ |
| Sheet-forming incubation | ○ | ○ | ○ |
| Rho-kinase inhibitor treatment | × | ○ | ○ |

Conditions of above-described each culture are shown in the following table.

**[Table 2]**

| | Comparative Example | Production Example 1 | Production Example 2 |
|---|---|---|---|
| FBS concentration | 10% | 10% | 20% |
| Culture days | 4 days | 2 days (48 hours) | 3 days (72 hours) |
| Seeding density (cells/cm²) | 2.1 × 10⁵ | 1.76 to 2.33 × 10⁶ | 1.76 to 2.33 × 10⁶ |
| Y27632 addition | Not performed | Day 1: performed | Day 1: performed |
| | | Day 2 or later: not performed | Day 2 or later: not performed |
| Freezing step | Not performed | Performed | Performed |

In Production Examples 1 and 2 with increased seeding densities, when, in total, a plurality of steps among an undifferentiated cell removing step and a freezing step having influence on cell viability were performed, sheet-forming was possible in a time shorter than that in Comparative Example.

Comparison of Comparative Example and Production Example 1 are as follows. When undifferentiated cell removal by heat treatment was performed alone and sheet-forming incubation was performed at a low seeding density (Comparative Example), the sheet formation required 4 days. On the other hand, when undifferentiated cell removal by Adcetris treatment was performed, then cryopreservation and thereafter thawing were performed, and the thawed material was seeded at a high density and subjected to sheet-forming incubation, and further Rho-kinase inhibitor treatment was performed during the sheet-forming incubation (day 1 only) (Production Example 1), sheet formation was possible in a shorter time. In addition, comparison of Comparative Example and Production Example 2 are as follows. Even when undifferentiated cell removal by heat treatment, sugar-free culture, and Adcetris treatment were performed, then cryopreservation and thereafter thawing were performed, and the thawed material was seeded at a high density and subjected to sheet-forming incubation, and Rho-kinase inhibitor treatment was performed during the sheet-forming incubation (day 1 only), sheet formation was possible in a shorter time.

### Example 2. Examination of culture days

For comparing influences of serum concentrations in sheet culture media, sheet-forming incubation was performed as in Production Example 2, except that FBS concentrations were 20% or 30%.

The results are shown in Fig. 1. When cultured in 20% FBS for 3 days, sheet-forming was possible. Thus, it was found that in conditions in Production Example 2, the FBS concentration was preferably 20% rather than 30%.

Further, using a sheet culture medium supplemented with 20% FBS, influence of a change in the number of culture days on qualities of sheet-shaped cell cultures was examined. When the number of culture days was 2 days, sheet-forming was observed in 7 experiments among 9 experiments. On the other hand, when the number of culture days was 3 days, sheet-forming was observed in all of 9 experiments. In addition, expression levels of Lin28 as an undifferentiated cell marker were examined. As a result, while the expression level was 0.022% at the beginning of culture, the expression level slightly increased to 0.033% at day 2 of the culture. However, at day 3 of the culture, the expression level decreased to 0.014%. In view of the results, it was clear that in the conditions in Production Example 2, when the FBS concentration was 20%, the number of days for sheet-forming incubation was preferably 3 days (72 hours). That is, the followings were found. When sheet-forming was performed by high-density seeding, sheet-forming was possible in a shorter time as compared to conventional methods. However, even when steps that were thought to reduce cell viability, such as an undifferentiated cell removing step and a cryopreservation step, were repeated, by setting the length of time of sheet-forming incubation to be longer (sheet-forming incubation days of at least 3 days (72 hours)), the following effects could be expected: a success rate of sheet formation was improved and the content of remaining undifferentiated cells was decreased.

### Example 3. Influence of filtering treatment step on sheet-forming

Influences of thawing cryopreserved cells and thereafter performing filtering treatment on sheet-forming were compared. In the above-described Production Example 2, whether or not there was a difference in quality between the following sheet-shaped cell cultures was examined using 3 lots of cardiomyocytes: when cryopreserved cells was thawed and seeded immediately thereafter (no filtration group) and when cryopreserved cells was thawed, subjected to filtering treatment using a 100-pm Falcon(R) cell strainer, and thereafter seeded (filtration group).

The results are shown in Figs. 2 and 3. When comparison between the no filtration group (group N) and the filtration group (group F) is made, it is found that there is no change in collected living cell numbers. On the other hand, viability was increased by performing the filtering treatment. In addition, in the filtering treatment group, it was observed that aggregated cells were decreased after sheet seeding. When troponin positive rates were measured before and after the filtering treatment, it was confirmed that the rate was 86% before the treatment and 86% after the treatment, that is, there was no change between before and after the treatment. In addition, no large difference in a Lin28 value as an undifferentiated cell rate was observed.

In the no filtration group, a perforations or breakage (a portion enclosed in a box in Fig. 3) was observed in 78% of sheet-shaped cell cultures. On the other hand, in the filtration group, occurrence of a perforation or breakage was reduced and 30% of sheet-shaped cell cultures had a perforation or breakage. That is, by removing, after thawing and before seeding of cells, dead cells from a cell population by filtering treatment, a clinically applicable sheet-shaped cell culture without perforation or breakage could be obtained with a high probability. That is, it was thought that when filtering treatment was performed after thawing and before seeding of cells, cell deaths during sheet-forming incubation could be reduced.

### Example 4. Influence of filtering treatment step on aggregate formation

In the filtering group and the no filtering group obtained in Example 3, seeded cells before sheet-forming were observed with a microscope. It was found that the number of gaps formed by formation of aggregates (cell aggregates) that were caused by dead cells per field of view (area of the field of view under 10-fold magnification was about 3 cm²) was decreased. One aggregate (cell aggregate) caused by dead cells counted as one gap. In the no filtering group, 19 aggregates (cell aggregates) were observed per field of view (area of the field of view under 10-fold magnification was about 3 cm²), and on the other hand, in the filtering group, 5 or less aggregates were observed (Fig. 4). As the amount of aggregates (cell aggregates) became smaller, more clinically applicable sheet-shaped cell cultures without perforation or breakage could be obtained.

### Example 5. Influence of seeding density and culture days on sheet forming

Production Example 3: Cardiomyocytes were differentiated from human iPS cells by inducing the differentiation, and thereafter undifferentiated cells were removed using anti-CD30 antibody-conjugated drug treatment described in WO 2016/072519 to obtain a cell population. Then, the cell population was cooled to -80°C at a cooling rate of -1°C/min using a program freezer, and thereafter cryopreserved in liquid nitrogen. Then, the cell population was thawed, subjected to filtering treatment using a 100-pm Falcon^{(R)} cell strainer, and thereafter subjected to sheet-forming incubation. The culture substrate used had been prepared on the day before cell seeding by adding 20% FBS-containing DMEM to a temperature responsive culture dish, and performing coating by incubation at 37°C overnight. In the sheet-forming incubation, cells were seeded, thereafter cultured under conditions at 37°C and 5% CO₂, and the culture medium was replaced daily. After the culture was completed, the cells were detached to form a sheet by decreasing the temperature. Only at day 1 of the sheet-forming incubation, a Rho-kinase inhibitor Y27632 was added to the sheet culture medium. The seeding density was 2×10⁵ cells/cm² to 1.2×10⁶ cells/cm², and the sheet-forming incubation days were 2 days or 3 days. Under the same conditions, this experiment was conducted using 5 lots of cardiomyocytes (n = 1 to 2).

The treatment conditions of each lot were as summarized in the following table.

**[Table 3]**

| | Lot A | Lot B | Lot C | Lot D | Lot E |
|---|---|---|---|---|---|
| Heat treatment | ○ | ○ | ○ | ○ | ○ |
| Sugar-free culture | ○ | ○ | ○ | ○ | ○ |
| Anti-CD30 antibody treatment | ○ | ○ | ○ | × | × |
| Plane culture | ○ | ○ | ○ | ○ | × |
| Freezing and thawing | ○ | ○ | ○ | ○ | ○ |

The results are shown in the following table and Fig. 5.

**[Table 4]**

| Culture days | Seeding density (cells/cm²) | Lot A | Lot B | Lot C | Lot D | Lot E |
|---|---|---|---|---|---|---|
| 2 days | 2.0 × 10⁵ | × | × | × | × | × |
| | 4.0 × 10⁵ | × | ○ | ○ | ○ | × |
| | 6.0 × 10⁵ | Δ○ | ○ | ○ | ○ | ○○ |
| | 8.0 × 10⁵ | ○○ | ○Δ | ○ | ○ | ○○ |
| | 1.0 × 10⁶ | ○○ | ΔΔ | ○ | ○ | × × |
| | 1.2 × 10⁶ | ○ | Δ | ○ | Δ | × |
| 3 days | 2.0 × 10⁵ | × | × | ○ | × | × |
| | 4.0 × 10⁵ | × | ○ | ○ | ○ | × |
| | 6.0 × 10⁵ | ○○ | ○○ | ○ | ○ | ○○ |
| | 8.0 × 10⁵ | ○○ | ○○ | ○ | ○ | ○○ |
| | 1.0 × 10⁶ | ○○ | ○○ | ○○ | ○ | ○○ |
| | 1.2 × 10⁶ | ○ | ○○ | ○○ | ○ | ○○ |

It was found that when a freezing and thawing step was performed at a low seeding density (2×10⁵ cells/cm²) as described in Example 2 of WO 2017/038562, sheet formation was impossible by 2 to 3 days of the sheet-forming incubation in most of the lots of cardiomyocytes. In addition, it was found that even when the seeding density was high (1.2×10⁶ cells/cm²), sheet formation was not possible at day 2 of the sheet-forming incubation in some cardiomyocytes. On the other hand, it was found that at day 3 of the sheet-forming incubation, sheet formation was possible without problems in all of the lots of cardiomyocytes within 6×10⁵ cells/cm² to 1.2×10⁶ cells/cm².

In addition, it was found that in cardiomyocyte lot E without plane culture, sheet formation was difficult as compared to other cell lots.

### Industrial Applicability

According to the present invention, when a graft such as a sheet-shaped cell culture is formed using, for example, cells that have been differentiated from pluripotent stem cells by inducing the differentiation, a high-quality graft can be obtained at a high probability. In particular, even when differentiation-induced cells having a decreased viability caused by various conditions required in a graft for use in clinical application are used, a high-quality graft can be formed easily.

## Claims

1. A method for producing a sheet-shaped cell culture, the method comprising:
(A) performing one single operation, or at least two different operations for removing undifferentiated cells in a cell population containing pluripotent stem cell-derived differentiation-induced cells, wherein said differentiation-induced cells are cardiomyocytes, wherein the one single operation is anti-CD30 antibody treatment, and wherein the at least two different operations comprise anti-CD30 antibody treatment; and
(B) seeding the cell population obtained in (A) on a culture substrate and performing graft-forming culture, wherein the cell population is seeded at a density reaching confluence, and wherein, in a sheet-forming incubation, a sheet culture medium used for sheet-forming incubation at day 1 contains a Rho-kinase inhibitor.

2. The method according to claim 1, wherein the pluripotent stem cells are iPS cells.

3. The method according to claim 1 or 2, further comprising, after (A), seeding and culturing the cell population on an adhesive culture substrate, and thereafter collecting the cell population.

4. The method according to any one of claims 1 to 3, wherein, after (A), the cell population is cryopreserved.

5. The method according to any one of claims 1 to 4, wherein the density reaching confluence is 0.40 to 2.33×10⁶ cells/cm².

6. The method according to any one of claims 1 to 5, wherein the graft-forming culture is performed for 2 to 3 days.

7. The method according to any one of claims 1 to 6, comprising:
(a) performing one single operation, or at least two different operations for removing undifferentiated cells in a cell population containing pluripotent stem cell-derived cardiomyocytes, wherein the one single operation is anti-CD30 antibody treatment, and wherein the at least two different operations comprise anti-CD30 antibody treatment;
(b) cryopreserving the cell population obtained in (a);
(c) thawing the cell population cryopreserved in (b);
(d) performing filtering treatment of the cell population thawed in (c); and
(e) seeding the cell population subjected to the filtering treatment in (d) on culture substrate at a density reaching confluence and performing sheet-forming incubation.

8. The method according to claim 7, wherein the cells are not proliferated during a period from (c) to (e).

9. The method according to claim 7 or 8, further comprising, after (a) and before (e), seeding and adhesively culturing the cell population on a culture substrate, and thereafter collecting the cell population.

## Patentansprüche

1. Verfahren zur Herstellung einer blattförmigen Zellkultur, wobei das Verfahren umfasst:
(A) Durchführen einer einzigen Operation oder mindestens zweier verschiedener Operationen zum Entfernen undifferenzierter Zellen in einer Zellpopulation, die von pluripotenten Stammzellen abgeleitete, durch Differenzierung induzierte Zellen enthält, wobei die durch Differenzierung induzierten Zellen Kardiomyozyten sind, wobei die eine einzige Operation eine Anti-CD30-Antikörperbehandlung ist und wobei die mindestens zwei verschiedenen Operationen eine Anti-CD30-Antikörperbehandlung umfassen; und
(B) Aussäen der in (A) erhaltenen Zellpopulation auf einem Kultursubstrat und Durchführen einer transplantatbildenden Kultur, wobei die Zellpopulation in einer Konfluenz erreichenden Dichte ausgesät wird, und wobei in einer blattbildenden Inkubation ein für die blattbildende Inkubation an Tag 1 verwendetes Blattkulturmedium einen Rho-Kinase-Inhibitor enthält.

2. Verfahren nach Anspruch 1, wobei die pluripotenten Stammzellen iPS-Zellen sind.

3. Verfahren nach Anspruch 1 oder 2, ferner umfassend nach (A) das Aussäen und Kultivieren der Zellpopulation auf einem adhäsiven Kultursubstrat und das anschließende Sammeln der Zellpopulation.

4. Verfahren nach einem der Ansprüche 1 bis 3, wobei nach (A) die Zellpopulation kryokonserviert wird.

5. Verfahren nach einem der Ansprüche 1 bis 4, wobei die Dichte bis zum Erreichen der Konfluenz 0,40 bis 2,33×10⁶ Zellen/cm² beträgt.

6. Verfahren nach einem der Ansprüche 1 bis 5, wobei die transplantatbildende Kultur 2 bis 3 Tage lang durchgeführt wird.

7. Verfahren nach einem der Ansprüche 1 bis 6, umfassend:
(a) Durchführen einer einzigen Operation oder mindestens zweier verschiedener Operationen zum Entfernen undifferenzierter Zellen in einer Zellpopulation, die aus pluripotenten Stammzellen abgeleitete Kardiomyozyten enthält, wobei die eine einzige Operation eine Anti-CD30-Antikörperbehandlung ist, und wobei die mindestens zwei verschiedenen Operationen eine Anti-CD30-Antikörperbehandlung umfassen;
(b) Kryokonservieren der Zellpopulation, die in (a) erhalten wurde;
(c) Auftauen der in (b) kryokonservierten Zellpopulation;
(d) Durchführen einer Filtrationsbehandlung der in (c) aufgetauten Zellpopulation; und
(e) Aussäen der Zellpopulation, die der Filtrationsbehandlung in (d) unterzogen wurde, auf Kultursubstrat in einer Dichte, die Konfluenz erreicht, und Durchführen einer blattbildenden Inkubation.

8. Verfahren nach Anspruch 7, wobei die Zellen während eines Zeitraums von (c) bis (e) nicht proliferiert werden.

9. Verfahren nach Anspruch 7 oder 8, ferner umfassend, nach (a) und vor (e), das Aussäen und die adhäsive Kultivierung der Zellpopulation auf einem Kultursubstrat und danach das Sammeln der Zellpopulation.

## Revendications

1. Un procédé de production d'une culture cellulaire en forme de feuille, le procédé comprenant :
(A) effectuer une opération unique, ou au moins deux opérations différentes pour éliminer des cellules indifférenciées dans une population cellulaire contenant des cellules induites par différenciation dérivées de cellules souches pluripotentes, dans laquelle lesdites cellules induites par différenciation sont des cardiomyocytes, dans laquelle l'opération unique est un traitement par anticorps anti-CD30, et dans laquelle les au moins deux opérations différentes comprennent un traitement par anticorps anti-CD30 ; et
(B) ensemencer la population cellulaire obtenue en (A) sur un substrat de culture et effectuer une culture de formation de greffons, dans laquelle la population cellulaire est ensemencée à une densité atteignant la confluence, et dans laquelle, dans une incubation de formation de feuilles, un milieu de culture de feuilles utilisé pour l'incubation de formation de feuilles au jour 1 contient un inhibiteur de la Rho-kinase.

2. Le procédé selon la revendication 1, dans lequel les cellules souches pluripotentes sont des cellules iPS.

3. Le procédé selon la revendication 1 ou 2, comprenant en outre, après (A), l'ensemencement et la culture de la population cellulaire sur un substrat de culture adhésif, puis la collecte de la population cellulaire.

4. Le procédé selon l'une des revendications 1 à 3, dans lequel, après (A), la population cellulaire est cryoconservée.

5. Le procédé selon l'une quelconque des revendications 1 à 4, dans lequel la densité atteignant la confluence est de 0,40 à 2,33×10⁶ cellules/cm².

6. Le procédé selon l'une quelconque des revendications 1 à 5, dans lequel la culture de formation du greffon est effectuée pendant 2 à 3 jours.

7. Le procédé selon l'une quelconque des revendications 1 à 6, comprenant :
(a) effectuer une seule opération, ou au moins deux opérations différentes pour éliminer les cellules indifférenciées dans une population cellulaire contenant des cardiomyocytes dérivés de cellules souches pluripotentes, dans laquelle la seule opération est un traitement par anticorps anti-CD30, et dans laquelle les au moins deux opérations différentes comprennent un traitement par anticorps anti-CD30 ;
(b) cryoconserver la population cellulaire obtenue au point (a) ;
(c) décongeler la population cellulaire cryoconservée en (b) ;
(d) effectuer un traitement de filtration de la population cellulaire décongelée en (c) ; et
(e) ensemencer la population cellulaire soumise au traitement de filtrage en d) sur un substrat de culture à une densité atteignant la confluence et effectuer une incubation de formation de feuilles.

8. Le procédé selon la revendication 7, dans lequel les cellules ne prolifèrent pas pendant une période allant de (c) à (e).

9. Le procédé selon la revendication 7 ou 8, comprenant en outre, après (a) et avant (e), ensemencer et cultiver de manière adhésive la population cellulaire sur un substrat de culture, puis collecter la population cellulaire.
